Europäisches Patentamt

European Patent Office

Office européen des brevets

(11) Veröffentlichungsnummer: **0 432 443 A1**

# (12) EUROPÄISCHE PATENTANMELDUNG

(21) Anmeldenummer: 90121162.3

(22) Anmeldetag: 06.11.90

(51) Int. Cl.5: **C12P 7/62**, C08G 63/06,
C12N 1/20, A61L 17/00,
A61L 31/00

Der Mikroorganismus ist bei der Deutschen Sammlung von Mikroorganismen und Zellkulturen Braunschweig unter der Nummer DSM 5630 hinterlegt worden.

(30) Priorität: 11.11.89 DE 3937649

(43) Veröffentlichungstag der Anmeldung:
19.06.91 Patentblatt 91/25

(84) Benannte Vertragsstaaten:
AT BE CH DE DK ES FR GB GR IT LI LU NL SE

(71) Anmelder: BOEHRINGER INGELHEIM KG

W-6507 Ingelheim am Rhein(DE)
(84) BE CH DE DK ES FR GR IT LI LU NL SE AT

Anmelder: BOEHRINGER INGELHEIM INTERNATIONAL G.M.B.H.

W-6507 Ingelheim am Rhein(DE)
(84) GB

(72) Erfinder: Steinbüchel, Alexander, Dr.
Stumpfe Eiche 44a
W-3400 Göttingen(DE)
Erfinder: Schlegel, Hans-Günther, Prof. Dr.
Görlitzer Strasse 35
W-3405 Bovenden(DE)
Erfinder: Timm, Arnulf
Rote Strasse 33
W-3400 Göttingen(DE)
Erfinder: Valentin,Henry, Dipl.-Biol.
Margueritenweg 6
W-3400 Göttingen(DE)

(54) Polyester auf der Basis von 4-Hydroxyalkansäuren und Verfahren zu ihrer Herstellung.

(57) Die vorliegende Erfindung betrifft Polyester auf der Basis von 4-Hydroxyalkansäuren, das Verfahren zu ihrer Herstellung, die dafür benötigten Mikroorganismen und die Verwendung der erfindungsgemäßen Polyester.

EP 0 432 443 A1

# POLYESTER AUF DER BASIS VON 4-HYDROXYALKANSÄUREN UND VERFAHREN ZU IHRER HERSTELLUNG

Die vorliegende Erfindung betrifft Polyester auf der Basis von 4-Hydroxyalkansäuren, Verfahren zu ihrer Herstellung und ihre Verwendung.

Insbesondere betrifft die Erfindung Polyester auf der Basis von $C_4$ bis $C_6$ 4-Hydroxyalkansäuren, besonders bevorzugt sind Polyester auf der Basis von 4-Hydroxybuttersäure.

Es ist bekannt, daß bestimmte Bakterien in der Lage sind, Polyester aus 3-Hydroxybuttersäure-Resten intrazellulär anzureichern. Derartige Polyester finden sich als Granula insbesondere in Zellen obligat aerober Mikroorganismen bzw. Bodenmikroorganismen. Diese Polyester liegen grundsätzlich als Polymere der D-(-)-ß-Hydroxybuttersäure vor (Dawes, E.A. und P.J. Senior, Arch. Microbiol. Physiol. 14 , 203-266, 1973).

Die bisher bekannten, über mikrobiologische Verfahren erhaltenen, biologisch abbaubaren Polyester aus gesättigten aliphatischen Hydroxymonocarbonsäuren (Alkansäuren), welche mit Hilfe von Alcaligenes eutrophus herstellbar sind, bestehen aus Copolymerisaten, die entweder aus 3-Hydroxybuttersäure (3HB) und 4-Hydroxybuttersäure (4HB) mit zufällig variierenden Anteilen davon bestehen (M. Kunioka et al., Appl. Microbiol. Biotechnol. 30 (6), 569-573, 1989) oder die ein zufälliges Gemisch aus zwei verschiedenen 3-Hydroxyalkansäuren enthalten (DE-OS 38 11 231, EP-A 288 908).

Bisher ist kein Herstellverfahren für isomerenreine Polyester aus 4-Hydroxyalkansäuren bekannt.

Resorbierbare Polyester finden großes Interesse im Bereich der medizinischen Verwendung. Aufgrund vielfältiger Anwendungen solcher Polymere, die zu nicht toxischen Produkten abgebaut werden, steigt das Interesse an resorbierbaren Polyestern mit genau definierter Struktur.

Es ist die Aufgabe der vorliegenden Erfindung neue, resorbierbare und biodegradierbare Polymere mit definierter Struktur herzustellen.

Gelöst wurde die Aufgabe durch Bereitstellung von Polyestern auf Basis von 4-Hydroxyalkansäuren, wobei bevorzugte Polyester isomerenreine Polyester davon sind.

Die Herstellung der erfindungsgemäßen Polyester erfolgt unter Verwendung von Mikroorganismen.

Ferner wurde gefunden, daß in 4-Stellung hydroxylierte, gesättigte aliphatische Monocarbonsäuren, vorzugsweise der Kettenlänge $C_4$ bis $C_6$,insbesondere 4-Hydroxybuttersäure, als Ausgangsstoffe zur Herstellung von sowohl isomerenreinen Polyestern, als auch von Polyestern, die durch einen Gehalt an > 40 mol %, >60 mol %, > 70 mol % oder > 90 mol % an 4-Hydroxyalkansäuren gekennzeichnet sind, verwendet werden können, wobei die Polymerisationsreaktion mit Hilfe von bestimmten Mikroorganismen und den davon hergestellten Mutanten erfolgen kann.

Eine derartige Synthese zu solchen Polyestern war nicht bekannt und war auch nicht zu erwarten, weil nach dem bisherigen Stand der Technik zum einen ausschließlich Copolyester erhalten werden konnten, die, bei Verwendung von 4HB als Kohlenstoffquelle einen variienden Anteil von 0 bis 37mol% 4HB enthalten (M. Kunioka et al., 1989, loc. cit.) und zum anderen nur die Biosynthese zu poly(3HB) bekannt ist.

Die mikrobielle Herstellung von isomerenreinen Polyestern und solchen mit > 40 mol % an 4-Hydroxyalkansäuren aus 4-Hydroxyalkansäuren mußte daher als nicht durchführbar betrachtet werden.

Überraschenderweise wurde gefunden, daß mit Mikroorganismen, von denen bekannt ist, daß sie 3HB verwerten und in Form von Poly(3HB) akkumulieren (z.B. Dawes, E.A. und P.J. Senior, 1973, loc. cit.) oder die auch 4HB verwerten können und bekannterweise nur solche Copolymere anreichen, die innerhalb eines begrenzten Bereichs aus einem zufälligen Anteil von Poly(4HB) bestehen (0 - 37 mol %), die erfindungsgemäßen Polyester aus entsprechenden 4-Hydroxyalkansäuren, insbesondere der Kettenlänge $C_4$ bis $C_6$, vorzugsweise 4-Hydroxybuttersäure, hergestellt werden können, wenn diesen Mikroorganismen im Wachstumsmedium eine entsprechende 4-Hydroxyalkansäure angeboten wird. Wenn derartige Mikroorganismen einem Mutationsverfahren unterzogen werden, können Mutantenstämme, die solche Polyester anreichern können, selektiv isoliert werden.

Somit sind auch die Mikroorganismen, die zur Synthese einer Poly(4-Hydroxylalkansäure) aus den entsprechenden 4-Hydroxyalkansäuren befähigt sind, Gegenstand der vorliegenden Erfindung, als auch solche, die durch Mutation dieser Mikroorganismen erhalten werden können.

Gemäß vorliegender Erfindung kann die Herstellung der erfindinngsgemäßen Polyester, durch ein Verfahren erfolgen, welches die folgende Schritte umfaßt:

1. Anreicherung und Selektionierung einer Reinkultur eines Mikroorganismus oder die Herstellung einer Reinkultur einer Mutante davon, welche zur Synthese eines aus 4-Hydroxyalkansäuren, vorzugsweise der Kettenlänge $C_4$ bis $C_6$, insbesondere aus 4-Hydroxybuttersäure, bestehenden Polyesters befähigt sind.

2. Züchtung der Reinkultur dieser Mikroorganismen, entweder im Batch-, fed-Batchverfahren (Handbuch der Biotechnologie, Präve, P.; Faust, U.; Sittig, W.; Sukatsch, D., Verlag Oldenburg, 2. Auflage 1984), im Zweischrittverfahren oder im kontinuierlichen Verfahren, in einem Kulturmedium, welches eine der genannten 4-Hydroxyalkansäuren, die gegebenenfalls als Salz, beispielsweise als Natrium- oder Kaliumsalz, vorliegen kann, enthält, derart, daß eine Anreicherung der entsprechenden Poly(4-Hydroxyalkansäure) erfolgt.

3. Isolierung des gebildeten Polyesters aus den Zellen der erfindungsgemäßen Mikroorganismen.

Für die Herstellung der erfindungsgemäßen Polyester eignen sich alle diejenigen Mikroorganismen, insbesondere Bodenmikroorganismen, vorzugsweise aeroben Bodenmikroorganismen, oder die davon aufgrund bekannter Mutations- und Anreichungsverfahren (Allgemeine Mikrobiologie, H.G. Schlegel, Thieme, Verlag 6. Auflage 1985) erhältlichen Mutanten-Stämme, die in der Lage sind, aus 4-Hydroxyalkansäuren die entsprechenden Polyester zu synthetisieren. Diejenigen Mikroorganismen, von denen bekannt ist, daß sie zur Synthese und Anreicherung von Poly(3-Hydroxybuttersäure) befähigt sind (Dawes, E.A. und P.J. Senior, 1973, loc. cit.), sind ganz bevorzugte Kandidaten.

Insbesondere sind solche Mikroorganismen und davon hergestellte Mutanten bevorzugt, die von den Genera Alcaligenes, Pseudomonas, Acetobacter, Nocardia, Actinomyces, Bacillus, Azotobacter, Aquaspirillum, Rhodospirillum, Paracoccus herstammen. Diese Mikroorganismen, von denen die Genera Alcaligenes und Pseudomonas ganz bevorzugte Ausgangsstämme für die Herstellung der erfindungsgemäßen Polyester sind, sind allgemein bekannt und beschrieben (Bergey's Manual of Determinative Bacteriology, H.R. Krieg, J. G. Holt; Williams & Wilkins, Baltimore/London Edition 1984)

Zur Anreicherung der Mikroorganismen erfolgt eine Inkubation in einem Medium mit bekannter Zusammensetzung an Kohlenstoffquellen, Mineralsalzen und Spurenelementen bei einer Temperatur zwischen 20 und 40°C, vorzugsweise in Bereich von 30°C, wie es beispielsweise in der EP-A 288 908 offenbart wird, in Gegenwart einer entsprechenden 4-Hydroxyalkansäure.

Die Herstellung der Mutanten der erfindungsgemäßen Mikroorganismen als Reinkultur, kann über folgende Mutations-, Anreicherungs-, Selektions- und Kultiverungsverfahren erfolgen:

Zur Auslösung der Mutation können die betreffenden Mikroorganismen nach Standardverfahren mit bekannten chemischen oder physikalischen Mutantionsauslösern behandelt werden. Beispielsweise können die Zellen für diesen Zweck in Gegenwart von Nitrosoharnstoffen, Nitrit oder Ethylmethansulfonat inkubiert oder energiereicher Strahlung ausgesetzt werden.

Die Anreicherung der Mutanten erfolgt in entsprechender Weise wie bei den Wildtyp-Zellen

Für die Selektion der Mutanten eignen sich Dichtegradientenzentrifugationen.

Es wurde jedoch gefunden, daß die Isolierung von Mutanten besonders gut gelingt, wenn sie über eine Zentrifugation in einem Percolldichtegradienten, beispielsweise nach Schübert, P. et al., J. Bact. 170, 5837-5847, 1988, durchgeführt wird.

Die Kultivierung der erfindungsgemäßen Mikroorganismen und der daraus erhaltenen Mutanten kann in üblichen Komplettmedien mit den bekannten synthetischen oder natürlichen Kohlenstoffquellen (C-Quellen) und mit den bekannten Stickstoffquellen und Mineralsalzen und Spurenelementen, wie sie beispielsweise in der EP-A 288 908 oder von Schübert, P. et al., 1988, loc. cit. offenbart werden, erfolgen.

Überraschenderweise wurde auch gefunden, daß mit den gefundenen Mikroorganismen schon beim ersten Schritt der Kultivierung eine der entsprechenden 4-Hydroxyalkansäuren dem Medium zugegeben werden kann und daß gleichzeitig mit der Zellvermehrung eine Synthese und Anreicherung der erfindungsgemäßen Polyester erfolgt. Dieses Verfahren hat den großen Vorteil, daß für eine weitere durchzuführende Züchtung zum Zweck der Anreicherung der erfindungsgemaßen Polyester schon präadaptierte Zellen zur Verfügung stehen und damit ein schnelleres Umschalten von Zellwachstum auf Speicherung der erfindungsgemäßen Polyester erfolgen kann.

Diese erfolgreiche Durchführung der Anzucht der erfindungsgemäßen Mikroorganismen in Gegenwart einer 4-Hydroxyalkansäure, entweder als einzige C-Quelle oder zusammen mit einer oder mehreren weiteren der bekannten C-Quellen, war nicht zu erwarten, da bei vergleichbaren Verfahren bisher nur bei einem dort notwendigen zweiten Kultivierungsschritt das Medium mit der maßgeblichen C-Quelle, beispielsweise mit einer Poly(3-Hydroxybuttersäure), supplementiert wurde (z.B. EP-A 288 908).

Die Dauer der Kultivierung der erfindungsgemäßen Mikroorganismen und der daraus erhaltenen Mutanten richtet sich nach den Kulturbedingungen, die sich in der Hauptsache nach der Temperatur, dem Sauerstoffgehalt des Mediums (aerobe Bedingungen) und nach dem Medium selbst (Menge der C-Quelle, der Mineralsalze, der Spurenelemente, pH-Wert) richten. Für die erfindungsgemäßen Mikroorganismen und die daraus erhaltenen Mutanten sind keine besonderen, dem Fachmann unbekannten Kulturbedingungen zu berücksichtigen und es können jene Bedingungen gewählt werden, wie sie beispielsweise in der EP-A 288 908 angegeben werden.

Die Menge der jeweils eingesetzten 4-Hydroxyalkansäure richtet sich nach dem jeweiligen Mikroorganismus bzw. dem daraus erhaltenen Mutanten-Stamm. Es kann jedoch von Konzentrationen im Bereich von

3

0.1% (wt/vol), entsprechend 1g 4-Hydroxyalkansäure pro Liter Kulturmedium, bis 10% (wt/vol), entsprechend 100g/Liter, insbesondere 0.2% (wt/vol) bis 5% (wt/vol) ausgegangen werden.

Die Zellernte kann allgemein während der log-Phase bis in die stationäre Phase erfolgen, vorzugsweise in der stationären Phase. Die Zellen können entweder nach einmaliger Kultur in ihrer Gesamtheit aus dem Medium gewonnen (batch- oder fed-Batch-Verfahren) oder laufend über eine kontinuierliche Kultur erhalten werden, beispielsweise durch übliche Zentrifugations- oder Filtrationsverfahren.

Die geernteten Zellen können, gegebenenfalls nach Waschen, beispielsweise mit einem Puffer, vorteilhafterweise mit einem Phosphatpuffer, insbesondere mit einem Natriumphosphatpuffer mit neutralem pH (pH 7.0), gefroren, lyophilisiert oder durch Sprühtrocknung behandelt werden.

Die Gewinnung der erfindinngsgemäßen Polyester kann nach bekannten Methoden erfolgen, bevorzugt wird die Lyse oder die Extraktion mit organischen Lösungsmitteln, insbesondere durch chlorierte Kohlenwasserstoffe, beispielsweise durch Chloroform oder Methylenchlorid.

Die erfindungsgemäßen Polyester sind als Thermoplaste leicht zu verarbeiten und vielseitig verwendbar. Beispielsweise in der Chirurgie für Gegenstände zum Wundverschluß (z.B. Nahtmaterial, Klammern), als Befestigungselemente für Knochen (wie z.B. Fixationsstifte, Platten, Schrauben, Dübel), als Trenn-, Füll- oder Abdeckmaterial (wie z.B. Gewebe, Vlies, Watte); in der Pharmazie (wie z.B. als Hilfsstoff, Trägermaterial, Freigabesysteme für Arzneimittel); zur Verkapselung und Mikroverkapselung von Substanzen und Wirkstoffen (wie z.B. Pflanzenschutzmitteln, Düngemitteln, Aromastoffen, Lebensmittelzusatzstoffen, Farbstoffen); zur Herstellung abbaubarer Verpackungsmittel (wie z.B. Folien, Hohlkörper wie Flaschen, Ampullen, Dosen, Beutel, Schachteln, Kisten); zur Herstellung anderer Gegenstände wie z.B. Filme, Fasern, Fäden) oder Formkörper des täglichen Bedarfs, bei denen eine Abbaubarkeit in der Umwelt erwünscht ist; als Ausgangsprodukt (Synthon) für andere chemische Verbindungen.

Die folgenden Beispiele sollen die Erfindung näher erläutern, wobei hervorzuheben ist, daß die unter den Beispielen angegebenen Verfahren grundsätzlich auch für andere Mikroorganismen, insbesondere der Gattungen Alcaligenes, Pseudomonas, Acetobacter, Nocardia, , Actinomyces, Bacillus, Azotobacter, Aquaspirillum, Rhodospirillum, Paracoccus, vorzugsweise solchen, von denen bekannt ist, daß sie zur Synthese und Anreicherung von Poly(3-Hydroxybuttersäure) befähigt sind (Dawes, E.A. und P.J. Senior, 1973, loc. cit.), anwendbar sind.

Beispiel 1

A. Herstellung der Mutante SK2813 aus Alcaligenes eutrophus Stamm JMP222 (DSM 5630, hinterlegt nach Budapester Vertrag am 03.11.1989)

Zellen von A. eutrophus Stamm JMP222 wurden in einem Komplexmedium (wie unter B. angegeben) in Gegenwart von 50 mM Nitrit für 10 Minuten bei 30° C inkubiert, danach in ein Mineralsalzmedium (wie unter B. angegeben) ohne Ammoniumchlorid mit 0,5% Fructose überführt (Schübert, P. et al., 1988, loc. cit.) und für 24 Stunden bei 30° C unter aeroben Bedingungen inkubiert. Aus der erhaltenen Zellsuspension wurden die gesuchten Mutanten durch Zentrifugation in einem Percolldichtegradienten (75% (vol/vol) Percoll [Sigma Chemical Co., St. Louis] in 150 mM NaCl, 0,2 ml Zellsuspension [ca. $10^8$ Zellen]) wie beschrieben (Schübert, P. et al., 1988, loc. cit.) isoliert. Die gesuchten Mutanten wiesen eine geringere Dichte auf und bandierten oberhalb der Wildtyp-Zellen. Der Gradient wurde fraktioniert wobei 0.1 ml Portionen auf MM-Fructose Platten mit 0.005% (vol/vol) $NH_4Cl$ plattiert wurden. Die Kolonien der gesuchten Mutanten erschienen weniger opak als diejenigen der Wildtyp-Zellen. Eine dieser Kolonien, als Mutante SK2813 bezeichnet, wurde für die weitere Aufarbeitung verwendet.

B. Kultivierung (batch-Kultur)

2 ml einer stationären Vorkultur der aus Schritt A. erhaltenen Mutante von SK2813 (50ml, 30° C, Schüttelkultur) in einem Komplexmedium, bestehend aus Beef extract (3g) und Pepton (5g), gelöst in einem Liter deionisiertem Wasser, wurden in 50 ml eines Mineralsalzmediums der Zusammensetzung

4

| Na$_2$HPO$_4$ x 12H$_2$O | 9.0g |
|---|---|
| KH$_2$PO$_4$ | 1.5g |
| NH$_4$Cl | 0.5g |
| MgSO$_4$ x 7H$_2$O | 0.2g |
| CaCl$_2$ x 2H$_2$O | 0.02g |
| Fe(III)NH$_4$-Citrat | 0.0012g, |

gelöst in einem Liter deionisiertem Wasser, welches 10 ml einer Spurenelementlösung enthielt. der Zusammensetzung

| Titriplex III | 500 mg |
|---|---|
| FeSO$_4$ x 7 H$_2$O | 200 mg |
| ZnSO$_4$ x 7 H$_2$O | 10 mg |
| MnCl$_2$ x 4 H$_2$O | 3 mg |
| H$_3$BO$_3$ | 30 mg |
| CoCl$_2$ x 6 H$_2$O | 20 mg |
| CuCl$_2$ x 2H$_2$O | 1 mg |
| NiCl$_2$ x 6 H$_2$O | 2 mg |
| Na$_2$MoO$_4$ x 2 H$_2$O | 3 mg, |

gelöst in einem Liter deionisiertem Wasser, supplementiert mit 0. 5% (wt vol) 4-Hydroxybuttersäure (Natriumsalz), inokkuliert und unter aeroben Bedingungen in einem Schüttelkolben bei 30°C für 48 h kultiviert.

C. Charakterisierung des erhaltenen Polyesters

Die aus Schritt B. erhaltenen Bakterien wurden über Zentrifugation geerntet, mit Natriumphosphatpuffer (pH 7.0) gewaschen, gefroren und lyophilisiert. Die Zellen wurden anschließend einer Methanolyse nach Brandl. H. et al., Appl. Environ. Microbiol. 54, 1977-1982, 1988, unterworfen. Der Gehalt und die Zusammensetzung des in der Zelle akkumulierten Polymers wurde über Gaschromatographie mit Methylestern von Hydroxyalkansäuren als Referenzsubstanzen bestimmt (Brandl. H. et al., 1988, loc. cit.).

Die Analyse des erhaltenen Polyesters ergab einen Polyestergehalt von 18.8 Gew.% der Trockenzellmasse. Das Polymer bestand ausschließlich aus Einheiten von 4-Hydroxybuttersäure (4HB)-Monomeren und lag somit als Homopolyester vor.

Beispiel 2

Es wurde wie unter Beispiel 1 angegeben verfahren, außer das anstatt des Mutantenstammes SK2813 der Wildtyp-Stamm A. eutrophus JMP222 verwendet wurde.

Die Analyse des vom Wildtyp-Stamm erhaltenen Polymers ergab einen Polyestergehalt von 21.5 Gew. % der Trockenzellmasse. Das Polymer bestand aus 92.1 mol% 4HB und 7.9 mol% 3-Hydroxybuttersäure (3HB).

Beispiel 3

Es wurde Beispiel 1 B. und C. wiederholt, außer das ein Wildtyp-Stamm von Pseudomonas sp. anstelle des Mutanten-Stammes SK2813 verwendet wurde.

Die Analyse des von dieser Pseudomonas-Mutante erhaltenen Polymers ergab einen Polyestergehalt von 18 Gew.% der Trockenzellmasse. Das Polymer bestand ausschließlich aus 4HB und lag somit als Homopolyester vor.

Beispiel 4

Die Kultivierung erfolgte in diesem Fall in einem Zweischrittverfahren unter Verwendung des Wildtyp-Stamms A. eutrophus Stamm JMP222. Im ersten Schritt wurde Stamm JMP222 in einem Komplexmedium (wie unter Beispiel 1 angegeben) für 48h bei 30°C unter aeroben Bedingungen inkubiert.

Im zweiten Schritt wurden diese Zellen über Zentrifugation geerntet und in einem Mineralsalzmedium (wie Beipiel 1), jedoch ohne NH₄Cl, suspendiert. Das Medium wurde mit 4-Hydroxybuttersäure (2%, wt/vol) supplementiert. Die Zellen, die aufgrund des Fehlens einer Stickstoffquelle nicht wachsen konnten, wurden bei 30°C für 48h unter aeroben Bedingungen inkubiert. Die Bakterien wurden über Zentrifugation geerntet, mit Natriumphosphatpuffer (pH 7.0) gewaschen, gefroren und lyophilisiert. Die Bestimmung des Gehalts und der Zusammensetzung des erhaltenen Polymers erfolgte wie unter Beispiel 1 ausgeführt. Der Gehalt an erhaltenem Copolymer betrag 42 Gew.% der Trockenzellmasse. Das Copolymer bestand aus 78.9 mol% 4HB und 21.1 mol% 3HB.

Beispiel 5

Es wurde Beispiel 1 B. und C. wiederholt, außer das Azotobacter sp. DSM 1721 anstelle von A. eutrophus JMP222 (Beispiel 2) verwendet wurde.

Der Gehalt an erhaltenem Copolymer betrug 14.6 Gew.% der Trockenzellmasse. Das Copolymer bestand aus 73.5 mol% 4HB und 26.5 mol% 3HB.

Beispiel 6

Von je einer 10 ml Vorkultur mit den folgenden beschriebenen und erhältlichen Bakterienstämmen Alcaligenes eutrophus A7 (Schwien, U, Schmidt, E., Appl. Environ. Microbiol. 44, 33-39, 1982), Alcaligenes eutrophus CH34 (Mergeay, M. et al., Arch. Int. Physiol. Biochem. 86, 440-441, 1978), Alcaligenes eutrophus JMP222 (Pemperton, J.M. et al., In: Plasmids of medical and commercial importance [K.N. Timmis & A. Pühler, eds.], Elsevier, Amsterdam, 1979), Alcaligenes eutrophus TF93 (ATCC 17697, DSM 531), Alcaligenes xylosoxidans ssp. denitrificans (Schmidt, T., Schlegel, H.G., FEMS Microbiol. Ecology 62, 315-328. 1989), Pseudomonas oxalaticus (DSM 1105) in einem Komplexmedium analog Beispiel 18. wurden jeweils 50 ml Mineralsalzmedium analog Beispiel 1B, welches 0.1 % (w/v) an 4-Hydroxyvaleriansäure (4HV) als alleinige Kohlenstoffquelle enthielt, in einem 300 ml Erlenmeyer-Kolben beimpft. Die Kulturen wurden bei 30°C geschüttelt. Nach erkennbarem Verbrauch der Kohlenstoffquelle wurde 4HV insgesamt vier- bis fünfmal ad 0.1 % (w/v) nachgefüttert bevor die Zellen geerntet, gewaschen und im Hinblick auf die Zusammensetzung des akkumulierten Polyesters analog Beispiel 1C. untersucht wurden.

Die Analyse der durch die jeweiligen Bakterien enthaltenen Terpolyester ergab die folgenden Resultate:

| Bakterienstamm | Zusammensetzung des | | |
| --- | --- | --- | --- |
| | Anteil Polyester an Zelltrockenmasse (%, w/w) | Polyesters (mol %) | |
| | | 3HB | 3HV | 4HV |
| A. eutrophus A7 | 16,4 | 29,3 | 67,2 | 3,4 |
| A. eutrophus CH34 | 45,0 | 15,0 | 82,2 | 2,7 |
| A. eutrophus JMP222 | 17,3 | 36,0 | 58,8 | 5,5 |
| A. eutrophus TF93 | 31,0 | 32,6 | 63,9 | 3,5 |
| A. xylosoxidans ssp.denitrificans | 33,7 | 16,1 | 80,8 | 3,1 |
| P. oxalaticus | 32,5 | 31,9 | 65,8 | 2,4 |
| 3HB = 3-Hydroxybuttersäure  3HV = 3-Hydroxyvaleriansäure  4HV = 4-Hydroxyvaleriansäure | | | |

Beispiel 7

In je 50 ml eines Komplexmediums gemäß Beispiel 1B. wurden die in Beispiel 6 genannten Bakterien bis zu hohen Zelldichten kultiviert, danach über Zentrifugation geerntet und wie in Beispiel 1C. beschrieben gewaschen und anschließend jeweils in ein gleiches Volumen Mineralsalzmedium analog Beispiel 1B., jedoch ohne Ammoniumchlorid ($NH_4Cl$), welches 0,5 oder 2,0% (w v) 4-Hydroxyvaleriansäure (4HV) als alleinige Kohlenstoffquelle enthielt, überführt. In diesem Medium wurden die Zellen bei 30°C für 48 Stunden geschüttelt. Der molare Anteil von 4HV in den nach diesem Verfahren erhaltenen Terpolyester war in der Regel höher als nach dem unter Beispiel 6 beschriebenen Verfahren und betrug bis zu 8 mol °%.

**Ansprüche**

1. Polyester auf der Basis von 4-Hydroxyalkansäuren.

2. Polyester nach Anspruch 1, dadurch gekennzeichnet, daß sie isomerenreine Polyester sind.

3. Polyester nach Anspruch 1, dadurch gekennzeichnet, daß sie einen Gehalt an > 40 mol °% einer 4-Hydroxyalkansäure enthalten.

4. Polyester nach Ansprüchen 1 bis 3, dadurch gekennzeichnet, daß die 4-Hydroxyalkalsäuren $C_4$ bis $C_5$ 4-Hydroxyalkansäuren sind.

5. Polyester nach Ansprüchen 1 bis 4, dadurch gekennzeichnet, daß sie aus 4-Hydroxybuttersäure Einheiten bestehen.

6. Verfahren zur Herstellung eines Polyesters nach einem der Ansprüche 1 bis 5 unter Verwendung von Mikroorganismen.

7. Verfahren nach Anspruch 6, dadurch gekennzeichnet, daß die Mikroorganismen Bodenmikroorganismen sind.

8. Verfahren nach Anspruch 7, dadurch gekennzeichnet, daß die Mikroorganismen ausgewählt sind aus den Gattungen Alcaligenes, Pseudomonas, Acetobacter, Nocardia. Actinomyces. Bacillus, Azotobacter. Aquaspirillum, Rhodospirillum, Paracoccus.

9. Verfahren nach einem der Ansprüche 6 bis 8, dadurch gekennzeichnet, daß die Mikroorganismen Mutanten sind.

10. Verfahren nach einem der Ansprüche 6 bis 9, dadurch gekennzeichnet, daß die Mikroorganismen und die daraus erhaltenen Mutanten in Gegenwart einer 4-Hydroxyalkansäure angezüchtet. angereichert oder kultiviert werden und nach der Zellernte die Polyester nach Ansprüchen 1 bis 5 aus diesen Mikroorganismen oder deren Mutanten gewonnen werden.

11. Verfahren nach einem der Ansprüche 6 bis 9, dadurch gekennzeichnet, daß die Mikroorganismen oder die daraus erhaltenen Mutanten zunächst in einem Komplexmedium angezüchtet werden. anschließend in ein Mineralsalzmedium in Gegenwart einer 4-Hydroxyalkansäure kultiviert werden und nach der Zellernte die Polyester gewonnen werden.

12. Verfahren nach Anspruch 11, dadurch gekennzeichnet, daß das Mineralsalzmedium kein Ammoniumchlorid enthält.

13. Polyester, herstellbar nach einem Verfahren gemäß Ansprüche 6 bis 12.

14. Verwendung eines Mikroorganismus gemäß einem der Ansprüche 6 bis 9 zur Herstellung von Polyestern nach Ansprüchen 1 bis 3.

15. Mikroorganismen zur Herstellung eines Polyesters nach Ansprüchen 1 bis 5.

16. Mikroorganismen nach Anspruch 15, dadurch gekennzeichnet, daß es Bodenmikroorganismen sind.

17. Mikroorganismen nach Anspruch 16, dadurch gekennzeichnet, daß sie ausgewählt sind aus den Gattungen Alcaligenes, Pseudomonas, Acetobacter, Nocardia, Actinomyes, Bacillus, Azotobacter. Aquaspirillum, Rhodospirillum, Paracoccus.

18. Der Mutanten-Stamm SK 2813 aus Alcaligenes eutrophus Stamm JMP222, DSM 5630, hinterlegt am 03.11.1989.

19. Verwendung eines Polyesters nach Ansprüchen 1 bis 5 in der Chirurgie, Pharmazie, zur Verkapselung und Mikroverkapselung von Substanzen und Wirkstoffen, zur Herstellung von Verpackungsmitteln und Formkörpern.

20. Formkörper, bestehend aus einem Polyester nach Ansprüchen 1 bis 5.

7

# EUROPÄISCHER RECHERCHENBERICHT

Nummer der Anmeldung

**EP 90 12 1162**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 304 293  (MITSUBISHI)<br>* Patentansprüche *<br><br>– – – | 1 | C 12<br>P 7 62<br>C 08 G 63 06<br><br>C 12 N 1 20 |
| A | FR-A-1 590 574  (UG. KUHLMANN)<br>* Zusammenfassung *<br><br>– – – – – | 1 | A 61 L 17 00<br>A 61 L 31 00 |

**RECHERCHIERTE SACHGEBIETE (Int. Cl.5)**

C 12 P
C 08 G

Der vorliegende Recherchenbericht wurde für alle Patentansprüche erstellt

| Recherchenort | Abschlußdatum der Recherche | Prüfer |
|---|---|---|
| Den Haag | 26 Februar 91 | DELANGHE L.L.M. |